# DEMANDE DE BREVET EUROPEEN

(11) **EP 1 493 433 A1**
(43) Date de publication de la demande: **05.01.2005**
(21) Numéro de dépôt: 04300394.6
(22) Date de dépôt: 23.06.2004
(51) Int. Cl.: A61K 9/14

(54) **Particules poreuses chargées en composé(s) actif(s) cosmétique(s) ou pharmaceutique(s)**

(30) Priorité: 26.06.2003 FR 0307747
(71) Demandeur: L'OREAL, 75008 Paris (FR)
(72) Inventeur: Simonnet, Jean-Thierry, 94240 Chachan (FR); Biatry, Bruno, 94300 Vincennes (FR); Saint-Leger, Didier, 92400 Courbevoie (FR); Leszczynski, André c/o Nony & Associes, 75008 Paris (FR)
(74) Mandataire: Le Coupanec, Pascale

(57) **Abrégé**

La présente invention concerne des particules poreuses individualisées caractérisées en ce qu'elles présentent un diamètre moyen, en volume, inférieur ou égal à 10 µm et une surface spécifique supérieure ou égale à 1 m²/g, et qu'elles comprennent au moins un composé cosmétiquement ou pharmaceutiquement actif au moins présent à l'intérieur desdites particules.

## Description

La présente invention concerne des particules poreuses individualisées ayant un diamètre moyen en volume inférieur ou égal à 10 µm et contenant au moins un composé cosmétiquement ou pharmaceutiquement actif, et leur utilisation notamment pour le transport et la libération dudit composé actif dans l'unité pilo-sébacée.

Pour augmenter l'efficacité de formulations à application topique, qu'elles soient de type cosmétique ou pharmaceutique, un certain nombre de méthodes ont déjà été proposées qui visent à améliorer la pénétration des molécules actives dans le stratum comeum formant la couche superficielle de la peau. On citera à titre d'exemples de ces méthodes, celles utilisant, comme véhicule de ces molécules actives, des liposomes, des nanocapsules, des émulsions H/E, des alcools courts, des glycols, ....

L'unité pilo-sébacée forme, au sein du stratum corneum, de l'épiderme et du derme, une invagination comprenant un follicule pileux et une glande sébacée. Elle est le siège d'une activité biologique et enzymatique importante ayant un effet majeur sur l'aspect de la peau. Parmi ces effets, on peut citer par exemple l'influence de la production de sébum sur le caractère gras ou sec de la peau, et l'influence de la croissance ou de la perte de croissance du poil ou du cheveu sur la pilosité de la peau. L'unité pilo-sébacée peut également faire l'objet d'un processus inflammatoire. Un tel processus peut avoir des causes diverses et être notamment lié à la présence de micro-organismes. Ce processus peut conduire ou contribuer à la manifestation d'un certain nombre de désordres cutanés tels que l'acné. En outre, l'unité pilo-sébacée constitue une voie de passage potentielle pour des agents destinés à agir sur les tissus cutanés profonds par exemple pour des agents de type anti-ride profond, de type amincissant, etc ...

La structure de cette unité pilo-sébacée, tant par sa morphologie avec la présence d'un poil, que par sa physiologie avec un flux continu de sébum, s'oppose naturellement à la pénétration et/ou la diffusion des composés actifs au sein et dans les profondeurs de cette structure.

Toutefois, des méthodes de ciblage de composés actifs dans l'unité pilo-sébacée ont déjà été proposées.

Ainsi, la demande EP 0 375 520 décrit l'utilisation de microsphères de polymères naturels ou synthétiques ou de corps gras de point de fusion supérieur à 50 °C, chargées au moins d'un produit actif, et dont au moins 80 % en poids de ces microsphères ont un diamètre compris entre 3 µm et 10 µm pour véhiculer préférentiellement le produit actif dans l'unité pilo-sébacée. Les microsphères décrites dans cette demande sont, soit des microsphères constituées de matériaux réticulés, soit des microsphères pleines chargées par solubilisation partielle de leur matériaux constitutifs et qui présentent une surface spécifique inférieure à 1m²/g. En outre, les procédés de préparation de microsphères décrits dans ce document qui comprennent l'encapsulation du composé actif soit à l'aide de solvants ayant une affinité suffisante vis-à-vis du matériau constitutif de la microsphère soit par la méthode dite émulsification-évaporation, ne permettent qu'un contrôle approximatif de l'homogénéité des microsphères obtenues et conduisent donc, outre leur faible capacité à charger le ou les composé(s) actif(s), à une variation de celle-ci ainsi qu'à une variation de leur capacité à relarguer le(s) composé(s) actif(s) dans l'unité pilo-sébacée.

La demande WO 02/07674 propose une méthode pour augmenter la pénétration d'un composé actif dans l'unité pilo-sébacée en utilisant une composition sous la forme de microsphères ou de liposomes ayant la propriété d'être introduite dans le follicule, et, d'y gonfler par mise en présence consécutive d'un agent de gonflement, de manière à y générer un passage dans le follicule. Toutefois, WO 02/07674 ne fournit pas d'exemple concret illustrant la méthode proposée et ne permet donc pas d'en vérifier l'efficacité.

Le brevet US 6,287,549 décrit une méthode d'épilation utilisant une composition comprenant des microparticules organiques chargées en agents chromophores, dans laquelle au moins 80 % en poids des microparticules utilisées ont une taille comprise entre 3 et 10 µm, afin de véhiculer l'agent chromophore dans l'unité pilo-sébacée. Ces microparticules peuvent être de natures diverses et chargées en chromophores soit lors de leur formation, soit par imprégnation de microcapsules déjà formées. Dans ces microparticules, les composés véhiculés ne sont pas des composés actifs en tant que tels car ils requièrent l'intervention d'un facteur extérieur pour pouvoir exercer un effet. En outre, l'exercice de cet effet ne nécessite pas leur libération des microparticules. Par ailleurs, ce document qui prévoit explicitement une étape optionnelle d'application d'une composition solubilisant les chromophores pour permettre leur libération des microparticules, ne suggère pas la possibilité d'un relargage passif et illustre même l'existence d'un préjugé à son encontre.

Le brevet US 4 690 825 décrit des véhicules constitués de particules poreuses de taille comprise entre 10 µm et 100 µm pour libérer de façon contrôlée, des ingrédients actifs. Ces particules sont préparées par copolymérisation de monomères à base de styrène ou de stéarate de vinyle et de divinylbenzène, ou de méthylméthacrylate et d'éthylène glycol diméthylméthacrylate, en présence d'un porogène qui est également l'ingrédient actif. Le produit obtenu risque donc de comporter des résidus de son procédé de préparation, ce qui est susceptible d'affecter son innocuité.

La demande WO 99/53904 décrit des capsules molles contenant une suspension huileuse ou une émulsion silicone/polyéthylène glycol et des microparticules sphériques poreuses préparées en particulier selon le brevet US 4 690 825 cité ci-dessus. Plus précisément, cette demande décrit des microparticules poreuses ayant un diamètre de particules moyen en poids de 20 µm chargées soit en rétinol, soit en acide ascorbique.

Enfin, le brevet US 6 387 995 décrit un procédé de fabrication d'un polymère adsorbant sous la forme de microparticules agglomérées c'est-à-dire non individualisées, de très faible masse volumique allant de 0,02 g/cm³ à 0,1 g/cm³, capable de piéger des composés lipophiles. La quantité de composé piégé dans les particules est négligeable par rapport à celle du composé piégé dans l'espace formé par les particules agglomérées.

On a maintenant découvert qu'il était possible de véhiculer au moins un composé actif et de le relarguer dans l'unité pilo-sébacée avec une efficacité accrue comparativement aux modes de réalisation discutés précédemment. Le moyen trouvé permet en outre d'améliorer l'encapsulation du composé actif, par rapport aux solutions proposées jusqu'à présent tout en présentant une innocuité particulièrement satisfaisante.

Selon un premier aspect, la présente invention concerne des particules poreuses individualisées ayant un diamètre moyen, en volume, inférieur ou égal à 10 µm et une surface spécifique supérieure ou égale à 1 m²/g, comprenant au moins un composé cosmétiquement ou pharmaceutiquement actif, au moins présent à l'intérieur desdites particules.

Selon un second aspect, la présente invention concerne une composition cosmétique ou pharmaceutique comprenant des particules telles que définies précédemment.

Par l'expression « particules poreuses », on désigne des particules ayant une structure comportant des pores. Cette structure poreuse peut notamment permettre au moins en partie l'incorporation d'un ou plusieurs agents actifs au sein desdites particules.

Cette structure peut être de type matriciel à l'image d'une éponge. Elle peut également être de type vésiculaire, c'est-à-dire que la particule présente une cavité interne délimitée par une paroi poreuse.

La porosité associée à la taille des particules est caractérisée quantitativement par leur surface spécifique. Les particules poreuses de l'invention présentent une surface spécifique mesurée selon la méthode BET supérieure ou égale à 1 m²/g.

Par l'expression « particules individualisées », on désigne des particules qui ne sont pas regroupées sous la forme d'agrégat ou d'agglomérat. Ces particules présentent en particulier une masse volumique supérieure ou égale à 0,15 g/cm³ et notamment allant de 0,2 à 5 g/cm³.

Par l'expression « composé cosmétiquement ou pharmaceutiquement actif », on entend, dans le cadre de la présente invention, un composé qui a par lui-même, c'est-à-dire ne nécessitant pas l'intervention d'un agent extérieur pour l'activer, une activité biologique. En outre, cette activité nécessite le fait que le composé soit en contact direct avec sa cible.

Les particules utilisées selon la présente invention dérivent de particules poreuses préformées, c'est-à-dire formées en l'absence du ou des composé(s) à encapsuler.

Au sens de la présente invention, on utilisera ci-après l'expression « particules chargées » pour désigner les particules selon l'invention de manière à les distinguer du matériau particulaire dont elles dérivent et qui ne contient pas de composé actif.

Les particules chargées selon l'invention ne comportent donc sensiblement pas de résidus liés au procédé de fabrication des particules dont elles dérivent, ce qui constitue bien entendu une amélioration en terme d'innocuité par rapport aux particules qui, pour pouvoir charger le composé actif, doivent être formées en sa présence. Par ailleurs, elles ne sont pas pleines.

Les particules de l'invention se caractérisent en particulier par une surface spécifique, mesurée par BET, élevée.

La méthode BET (BRUNAUER-EMMET-TELLER) est une méthode bien connue de l'homme du métier. Elle est notamment décrite dans « The journal of the American Chemical Society », vol.60, page 309, février 1938 et correspond à la norme internationale ISO 5794/1 (annexe D). La surface spécifique mesurée selon la méthode BET correspond à la surface spécifique totale, c'est-à-dire qu'elle inclue la surface formée par les pores.

Selon un mode de réalisation particulier, les particules de l'invention présentent une surface spécifique mesurée par BET, allant notamment de 2,5 à 1000 m²/g, en particulier de 3 à 750 m²/g, plus particulièrement supérieure ou égale à 300 m²/g, voire supérieure ou égale à 500 m²/g.

Comme mentionné précédemment, les particules selon la présente invention ont un diamètre moyen en volume inférieur ou égal à 10 µm.

En effet, de telles particules peuvent pénétrer dans le follicule sébacé par application d'une force mécanique. Cette force mécanique provient généralement d'un massage qui, outre la poussée qu'elle exerce, génère un effet de pompe au niveau du follicule.

Les particules atteignent ainsi progressivement le canal folliculaire dans lequel le composé actif qu'elles portent, peut alors diffuser, et accéder éventuellement aux tissus environnant le canal folliculaire. En revanche, le support, qui constitue la particule est ensuite rejeté grâce à l'écoulement de sébum et/ou à la pousse du poil permettant ainsi d'éviter toute réaction indésirable de l'organisme vis-à-vis du composé solide constituant les particules.

Il faut noter que des particules ayant un diamètre supérieur à 10 µm, même avec application d'une force mécanique similaire, restent pour la plupart localisées sur la surface de la peau sans y pénétrer et ne peuvent donc relarguer le composé actif que sur la couche cornée.

Selon un mode de réalisation particulier de l'invention, les particules ont un diamètre moyen en volume supérieur ou égal à 0,1 µm et en particulier allant de 0,5 à 8 µm.

Les particules utilisées selon l'invention sont des particules, notamment sphériques, poreuses, de dimension moyenne en nombre pouvant aller de 0,1 à 50 µm, notamment de 0,1 à 20 µm et tout particulièrement de 0,5 à 10 µm.

Par "dimension moyenne en nombre" on désigne la dimension donnée par la granulométrique statistique à la moitié de la population, dite D50.

Selon une variante de l'invention, les particules se caractérisent par leur homogénéité granulométrique. En particulier, elles présentent un indice de polydispersité, IP, allant de 1 à 4, et notamment inférieur ou égal à 3. Cet indice de polydispersité est défini comme le rapport D(4,3)/D(3,2), dans lequel D(4,3) désigne le diamètre moyen en volume et D(3,2) désigne le diamètre moyen en surface. Ces deux valeurs sont communément mesurées à l'aide de granulomètres à diffraction laser tel que celui commercialisé sous la dénomination de « Mastersizer 2000 » par la société MALVERN.

Les particules poreuses de l'invention peuvent avoir des formes variées, notamment globulaire et en particulier sensiblement sphérique.

Les particules poreuses dont dérivent les particules chargées selon l'invention sont généralement constituées de matériaux totalement insensibles, en termes notamment de solubilisation et de plastification, au procédé d'encapsulation des composés actifs, en particulier dans le cas où celui-ci fait intervenir pour l'imprégnation un solvant organique.

Ces particules peuvent être de type organique, inorganique ou mixte et se présentent le plus souvent sous forme de poudre ayant en particulier une volatilité réduite.

Comme particules poreuses de type organique, on citera à titre d'exemple les particules de Nylon 6, Nylon 6-6, Nylon 12 ou Nylon 6-12 telles que celles commercialisées par la société ATOFINA sous le nom générique d'« Orgasol », les particules de polyméthacrylate de méthyle (PMMA) telles que celles commercialisées sous la dénomination de « Covabead® » par la société WAKER.

Dans un mode de réalisation particulier de l'invention, les particules utilisées sont choisies parmi les particules de Nylon citées ci-dessus.

Selon une variante de l'invention, les particules selon l'invention sont de nature inorganique.

A titre illustratif des matériaux inorganiques utilisables dans les particules selon l'invention, on peut citer la silice, la silice alumine, l'hydroxyapatite, le dioxyde de titane, la séricite, le mica, le carbonate ou l'hydrocarbonate de magnésium, les oxydes d'aluminium de type alumine et les silicates mixtes, comme les aluminosilicates, et leurs mélanges.

Parmi les particules minérales poreuses susceptibles de convenir à l'invention on peut citer les microsphères de silice creuses, les microsphères de silice poreuses et les microcapsules de verre ou de céramique.

En particulier, les particules minérales poreuses convenant à l'invention peuvent être choisies parmi :
- les particules de silice comme celles commercialisées par ASAHI GLASS sous le nom de « Sunsphere H series », et par SUZUKI OIL AND FAT sous le nom de « God Balls »,
- les particules d'hydroxyapatite comme celles commercialisées par MERCK (sous la référence 1051990010 - granulométrie 15 µm), ou bien celles commercialisées par les sociétés LABORATORY SKIN CARE ou ASAHI GLASS et SEKISUI sous les noms respectifs « Hydroxyzomes » (LSC et Asahi Glass), AP20C et AP12C (SEKISUI), d'« ASP® » par la société SEKISUI PLASTICS.
- les particules de silice-alumine telles que celles commercialisées sous la dénomination de « Zeeosphere® » par la société 3M,
- les particules de dioxyde de titane telles que celles commercialisées par la société ISHIHARA, et
- les particules composées de mélange de ces minéraux.

Dans un mode de réalisation de l'invention, les particules utilisées sont en particulier choisies parmi des particules de silice et d'hydroxyapatite.

Les particules poreuses utilisées dans la présente invention peuvent être également constituées de matériaux composites organiques et inorganiques.

Les particules chargées selon la présente invention comprennent au moins un composé cosmétiquement ou pharmaceutiquement actif, ledit composé étant au moins présent à l'intérieur desdites particules poreuses. Le composé actif peut être également présent à la surface des particules chargées, mais dans un tel cas, il est généralement majoritairement présent à l'intérieur desdites particules.

Le rapport pondéral entre le ou les composés actifs et les particules poreuses non chargées en composé(s) actif(s) est généralement de 1/1000 à 10/1, en particulier de 1/100 à 1/1.

Les actifs utilisés peuvent être des composés bien connus de l'homme du métier. Ce sont généralement des agents actifs habituels dans le domaine cosmétique ou dermatologique.

Les composés actifs peuvent être hydrophiles ou lipophiles. Selon une variante particulière de l'invention, les particules chargées comprennent au moins un composé actif lipophile. Elles peuvent également comprendre au moins un composé actif hydrophile, ce dernier pouvant être suffisamment solubilisé par des composés amphiphiles présents dans le sébum pour permettre son relargage.

Les composés actifs considérés ci-après sont indifféremment hydrophiles ou lipophiles.

Parmi les composés actifs, on peut citer notamment :
- les agents antibactériens tels que le triclosan, l'IPBC (iodo-3 propynyl-2 butyl carbamate), le chlorure de benzalkonium, la chlorexhidine, le totarol® (extrait de plantes comprenant du totara-8,11,13-trien-13-ol),...
- les agents antifongiques tels que la piroctone olamine, le zinc pyrithione, le climbazole, le rilopirox, le kétoconazole, l'itraconazole ...,
- les agents sébo-régulateurs tels que les dérivés d'iminodibenzyle ou de fluorène tels que décrits dans le brevet US 6 355 687, les dérivés d'amines secondaires substituées tels que décrits dans le brevet US 6 355 686, les dérivés d'acide glucuronique et de glucosamine et leurs sels, tels que décrits dans la demande de brevet EP 1 219 296, les associations de niacinamides avec un alkyle ou un ester d'alcényle en C₁₁-C₃₀ d'acide salicylique tels que décrits dans la demande de brevet WO 02/067 889,
- les agents sébo-stimulateurs tels que la DHEA et ses dérivés synthétiques ou naturels, les dérivés α-hydroxylés de vitamine D1 tels que ceux décrits dans le brevet US 6 369 099,
- les agents kératolytiques tels que l'acide salicylique et ses dérivés, comme plus particulièrement l'acide n-octanoyl-5 salicylique, les alpha hydroxyacides tels que ceux par exemple de l'acide glycolique, lactique ou malique, et la résorcine,
- les agents de traitements de l'acné tels que le rétinol et ses dérivés, l'acide rétinoïque et ses isomères tout trans ou 13 cis, le peroxyde de benzoyle, les inhibiteurs du cytochrome P450 tels que décrits dans le brevet US 6 399 774 et leurs dérivés et l'acide azélaïque,
- les antibiotiques de structure macrolidique ou non, les composés d'Avermectine tels que décrits dans le brevet US 6 399 652, l'ester de 2,6-diisopropyl-phényl et l'acide de [(2,4,6-triisopropyl-phényl)-acétyl] sulfamique ou d'un sel de celui-ci, comme inhibiteur de la synthèse de cholestéryle et d'esters de cire, tel que décrit dans la demande de brevet WO 01/56556,
- les agents inhibiteurs de la chute du cheveu ainsi que les agents stimulateurs de la pousse du cheveu tels le minoxidil, la biotine, le finastèride, le 2,4 dipirymidine N-oxyde, le panthénol et leurs dérivés, la flavanone T, ou plus généralement tout extrait végétal, possédant une activité anti 5 alpha réductase de type I ou II,
- les agents inhibiteurs de la pousse du cheveu ou du poil tels que les sérines protéases décrites dans le brevet US 6 407 056, l'acide caféique, la quercétine, le gallate de propyle, l'acide norhydroguaiarétique ou NDGA, l'indométhacine, l'hydrochlorure d'eflornithine, les extraits végétaux tels décrits dans le brevet US 6 171 595 comme les extraits de clou de girofle, de bouton de rose, de pimprenelle (burnet), de gambir ..., les composés décrits dans le brevet US 6 075 052, le tétramisole, l'ortho vanadate de sodium, le levamisole, le chromoglycate disodique, le nitrate de vanadium et le nitrate de gallium tel que décrit dans le brevet US 6 020 006, ainsi que les composés décrits dans les brevets US 4 885 289, US 4 720 489, US 5 132 293, US 5 096 911, US 5 095 007, US 5 143 925, US 5 328 686, US 5 440 090, US 5 364 885, US 5 411 991, US 5 648 394, US 5 468 476, US 5 475 763, US 5 455 608, US 5 674 477, US 5 728 736 et 5 652 273 et dans les demandes de brevet WO 94/27586, WO 94/27563 et WO 98/03149. On pourra également utiliser les extraits de génévrier tels que décrits dans le brevet US 6 375 948,
- les agents anti-pelliculaires tels que la pyrithione de zinc,
- les agents antioxydants tels que le butylhydroxytoluène (BHT), les caroténoïdes tels que le β-carotène, le lycopène, la canthaxanthine, l'ubiquinone, le tétra hydroxycinnamate de dibutyl pentaérythrityle, la vitamine E, le trolox, la vitamine C et ses dérivés,
- les agents astringents et agents réducteurs des pores tels que ceux décrits dans la demande de brevet WO 02/32392,
- les agents anti-transpirants tels que les sels d'aluminium et de zirconium,
- les vitamines, autres que celles mentionnées précédemment et telles que la vitamine B3, la vitamine K, la vitamine H, la vitamine PP, la vitamine D, la vitamine B6 et leurs dérivés, et
- les agents anti-inflammatoires tels que l'α-bisabolol, le glycyrrhizinate de dipotasium, l'acide glycyrrhétinique et ses dérivés, l'acide ellagique, l'acide ursolique, l'ibuprofène, le naproxen, le fénoprofen, le carprofen, le kétoprofène, les anti-inflammatoires stéroïdiens tels que la cortisone, la pregnénolone, le désonide et les mélanges d'alcanolamines et de tyrosine tels que ceux décrits dans la demande de brevet EP 1 192 939.

On peut en particulier citer tous les actifs connus pour leur activité sur le vieillissement de la peau comme les agents keratolytiques ou prodesquamants, par exemple les α-hydroxy-acides, les β-hydroxy-acides, les α-céto-acides, les β-céto-acides, les rétinoïdes et leurs esters, le rétinal, l'acide rétinoïque et ses dérivés.

On peut citer également les vitamines, telles que les vitamines C, B3 ou PP, B5, E, K1, et les dérivés de ces vitamines et notamment leurs esters ; les agents anti-radicaux libres ; la DHEA et ses dérivés ; le coenzyme Q10 ; les agents blanchissants et dépigmentants comme l'acide kojique, les dérivés de para-aminophénols, l'arbutine et leurs dérivés, et leurs mélanges.

On peut encore citer les actifs utiles pour les peaux grasses, tels que les sels de zinc et en particulier le gluconate de zinc ; les antibactériens comme l'acide salicylique, le triclosan, le lipacide, l'extrait de clou de girofle, l'octopirox, l'hexamidine ; les actifs anti-acné.

La quantité d'actif introduit dans les particules dépend de l'effet recherché. Les actifs peuvent être présents dans les particules poreuses en une quantité en matière active allant de 1 à 50 % en poids, notamment de 2 à 40 % en poids, et en particulier allant de 5 à 30% en poids par rapport au poids total des particules une fois chargées.

Les particules chargées de la présente invention sont préparées selon des méthodes conventionnelles, en particulier par imprégnation.

En particulier, les particules chargées selon l'invention sont obtenues par imprégnation de particules poreuses préformées avec au moins un composé actif. Avantageusement, ce protocole ne requiert pas la présence d'un porogène.

A titre d'exemple, le procédé d'imprégnation consiste à préalablement solubiliser le ou les composés à encapsuler dans un solvant adapté et en quantité nécessaire et suffisante pour imprégner les particules, puis à mettre ce mélange en présence de particules poreuses conformes à l'invention. Le solvant est ensuite évaporé jusqu'à l'obtention d'une poudre sèche. La poudre ainsi obtenue ne contient généralement qu'une très faible proportion en solvant résiduel, de l'ordre de 1/10 ppm.

Comme solvants pouvant être utilisés dans un tel procédé d'imprégnation, on peut citer notamment l'acétone, l'éthanol, l'isopropanol, le dichlorométhane, l'acétate d'éthyle ... Bien entendu, le choix du solvant est effectué en tenant compte de la nature des composants des particules poreuses et des composés à encapsuler.

Dans le cas où le composé à encapsuler se présente sous la forme d'un liquide, on pourra directement mettre en contact celui-ci avec les particules poreuses sans l'ajout d'un solvant annexe.

L'homme du métier veillera à choisir les conditions d'imprégnation de façon à obtenir une poudre sèche.

Les particules chargées de l'invention permettent l'administration spécifique dudit ou desdits composé(s) cosmétiquement ou pharmaceutiquement actif(s) dans l'unité pilo-sébacée.

Ces particules peuvent être introduites dans différentes formulations cosmétiques ou pharmaceutiques destinées à une application topique.

La présente invention se rapporte donc en outre une composition cosmétique ou pharmaceutique comprenant des particules chargées telles que définies précédemment.

Bien entendu, ladite composition peut ne comprendre qu'un seul type de particules telles que définies précédemment ou bien un mélange de telles particules.

Généralement, la composition contient de 0,1 à 50 % en poids et en particulier de 0,2 à 20 % en poids de particules telles que définies précédemment par rapport au poids total de la composition.

La composition selon l'invention peut comprendre en outre :
- au moins un composé cosmétiquement ou pharmaceutiquement actif destiné à agir essentiellement hors de l'unité pilo-sébacée, et/ou
- au moins un additif cosmétiquement ou pharmaceutiquement acceptable, et/ou
- un support galénique qui peut être de tout type approprié.

On entend désigner par « support » tout mode de véhicule compatible avec une utilisation cosmétique ou pharmaceutique, à savoir de type liquide comme de l'eau, un solvant hydroalcoolique, de l'huile ou un de leurs mélanges, ou de type solide comme une cire par exemple.

On veillera cependant à ce que le composé cosmétiquement ou pharmaceutiquement actif supplémentaire, l'additif et le support éventuels n'entraînent pas le relargage du composé actif dans la composition.

Les compositions de l'invention peuvent contenir en outre des adjuvants classiques tels que des colorants, des pigments, des parfums, des conservateurs, des filtres solaires physiques et chimiques, des séquestrants, des actifs liposolubles ou hydrosolubles, des hydratants tels que les polyols et notamment la glycérine, des ajusteurs de pH (acides ou bases).

Selon un mode de réalisation particulier, les compositions de l'invention sont sensiblement exemptes d'agents tensioactifs.

La composition cosmétique ou pharmaceutique peut se présenter sous la forme de lotions, d'émulsions H/E ou E/H, de gels aqueux ou hydroalcooliques, ou bien encore sous forme anhydre telle que des sticks, des sprays ou des poudres compactes ou libres.

Les compositions de l'invention peuvent être des compositions pour le soin, l'hygiène ou le maquillage de la peau du corps ou du visage, ou des matières kératiniques telles que les ongles, les cils, les sourcils ou les cheveux.

Elles peuvent être par exemple destinées à un usage capillaire et être en particulier des shampoings, des après-shampoings, des lotions capillaires en particulier de soins.

Ces compositions peuvent être également un produit démaquillant, notamment une huile, un gel, ou une lotion démaquillante ou moussante.

Elles peuvent également être des sticks de maquillage tels que des rouges à lèvres, ou des sticks d'hygiène telles que des déodorants.

Elles peuvent être encore un produit de maquillage notamment de type fond de teint, crème teintée, mascara ou eye-liners.

Les exemples présentés ci-après sont donnés à titre illustratif et sans caractère limitatif.

### FIGURE :

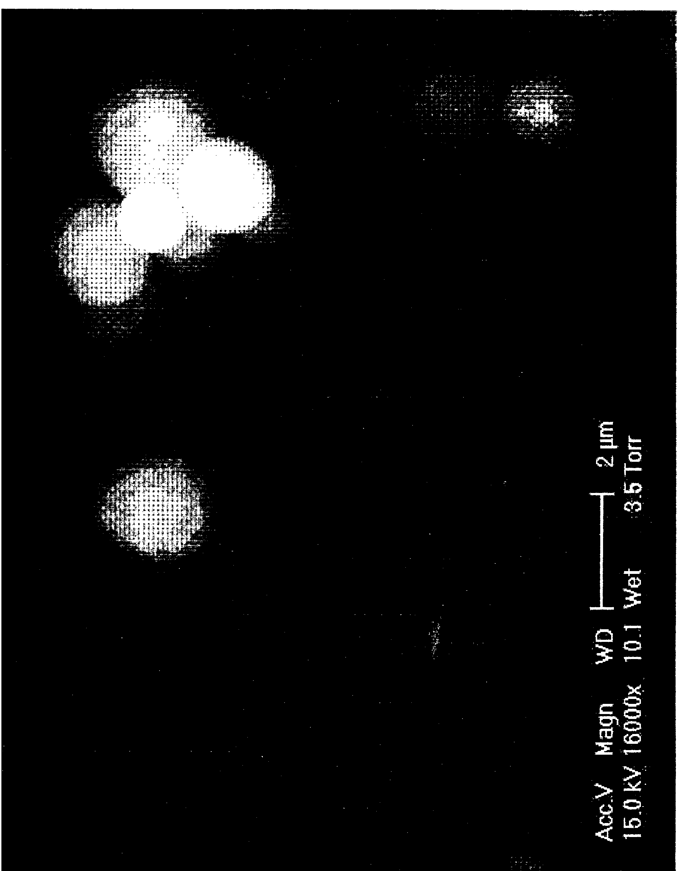

Figure 1 : Photographie électronique constituée de particules de silice contenant du triclosan conforme à l'invention.

### Exemple 1 : Préparation de particules minérales contenant un composé actif et une formulation dans une composition cosmétique

Une solution contenant 20 g d'acide salicylique, 1 L d'acétone et 200 g de silices poreuses (Sunsphere H33) a été réalisée. Cette solution est maintenue sous agitation à température ambiante jusqu'à la solubilisation complète de l'actif. La solution est ensuite transférée dans un ballon et l'évaporation de l'acétone est effectuée sous rotavapor à 40 °C. On obtient ainsi après évaporation complète du solvant une poudre constituée de particules de silice poreuses dont les pores comprennent de l'acide salicylique sous forme solide. La composition en poids des particules obtenues est de 10 % en poids en acide salicylique et 90 % en poids de silice par rapport au poids total des particules.

Nous avons formulé des compositions cosmétiques contenant les particules selon l'invention.

### a) Poudre libre matifiante

| **Poudre libre matifiante** | **Composition témoin** **(% en poids)** | **Composition selon l'invention** **(% en poids)** |
|---|---|---|
| Talc | 87 | 69 |
| Acide salicylique | 2 | --- |
| Diméthicone | 7,5 | 7,5 |
| Stéarate de magnésium | 2,5 | 2,5 |
| Particules selon l'invention (10 % acide salicylique - 90 % silice) | --- | 20 |
| Conservateurs | 1 | 1 |

On a évalué la stabilité de la composition selon l'invention, c'est à dire comprenant 20 % de particules selon l'invention par rapport à une composition témoin ne contenant pas de particules conformes à l'invention. Les poudres ont été conditionnées dans un boîtier de 10 g. Les boîtiers furent ensuite placés pour une période de 2 mois dans une étuve dont l'humidité relative est fixée à une valeur de 80 % et la température fixée à une valeur de 45 °C. L'aspect macroscopique des poudres après stockage en étuve est ensuite évalué optiquement. Les résultats sont reportés dans le tableau suivant :

| **Composition** | **Témoin** | **Selon l'invention** |
|---|---|---|
| Aspect | Poudre rosâtre, présentant des taches rose foncé | Poudre blanche ne présentant aucune coloration en surface et dans la masse |

Ces résultats, montrent qu'après un stockage prolongé en atmosphère humide la composition selon l'invention ne présente aucune coloration inesthétique.

### Exemple 2

Deux compositions contenant un composé actif lipophile, l'acide n-octanoyl-5 salicylique, à savoir respectivement, un gel contenant des particules poreuses de Nylon de 4 µm (« Orgasol »®), objet de l'invention, et une émulsion H/E de même granulométrie sont comparées en terme d'efficacité de ciblage de l'unité pilo-sébacée. Le taux de principe actif, l'acide n-octanoyl-5 salicylique, est identique dans les deux types de composition, et fixé à 0,3 % en poids.

### Les compositions testées

| Composition 1 (selon l'invention) | |
|---|---|
| Polyacryloyldiméthyltaurate d'ammonium | 0,50 g |
| Particules poreuses de Nylon-12* | 4,70 g |
| Acide n-octanoyl-5 salicylique | 0,30 g |
| Poloxamer 338 | 0,25 g |
| Eau déminéralisée | 94,25 g |

| | |
|---|---|
| *Les particules poreuses de Nylon-12 sont commercialisées sous la dénomination d'« Orgasol 2002 UD Nat cos » par la société ATOFINA. | |

| Composition 2 (émulsion H/E comparative) | |
|---|---|
| Gomme de xanthane | 0,10 g |
| Stéarate de glycéryle | 1,00 g |
| Hydroxyde de sodium | 0,10 g |
| Alcool cétylique | 2,00 g |
| Octyldodécanol | 9,00 g |
| Glycérol | 3,00 g |
| Polyisobutène hydrogéné | 2,00 g |
| Eau | 71,95 g |
| Acide n-octanoyl 5 salicylique | 0,30 g |
| Huile de paraffine | 5,00 g |
| Carbomer | 0,30 g |
| Stéarate de PEG-100 | 1,00 g |
| Polysorbate 60 | 4,00 g |
| Méthylparaben | 0,25 g |

L'étude a été effectuée sur huit personnes volontaires qui possèdent une peau grasse présentant des pores dilatés sur le front.

Pour chaque sujet, après avoir soigneusement nettoyé le visage au savon, on applique sur la moitié latérale gauche ou droite du front 4 mg/cm² de la composition à tester, puis on masse la zone traitée pendant 1 minute et on laisse sécher pendant 15 minutes. Cette application est renouvelée pendant 4 jours dans les mêmes conditions (soit une durée totale de traitement de 5 jours avec une seule application quotidienne).

Au jour 6, on effectue un prélèvement épidermique sur chaque sujet par strip au cyanoacrylate, en appliquant sur le front de chaque individu une lame de verre sur laquelle a été déposée une goutte de cyanoacrylate, puis après séchage, en retirant ladite lame qui entraîne ainsi un échantillon épidermique.

Les follicules et les comédons sont ensuite prélevés à partir desdits échantillons et leur contenu est extrait dans du méthanol. Le taux de composé actif est quantifié par HPLC.

Les résultats sont présentés dans le tableau 1 ci-après.

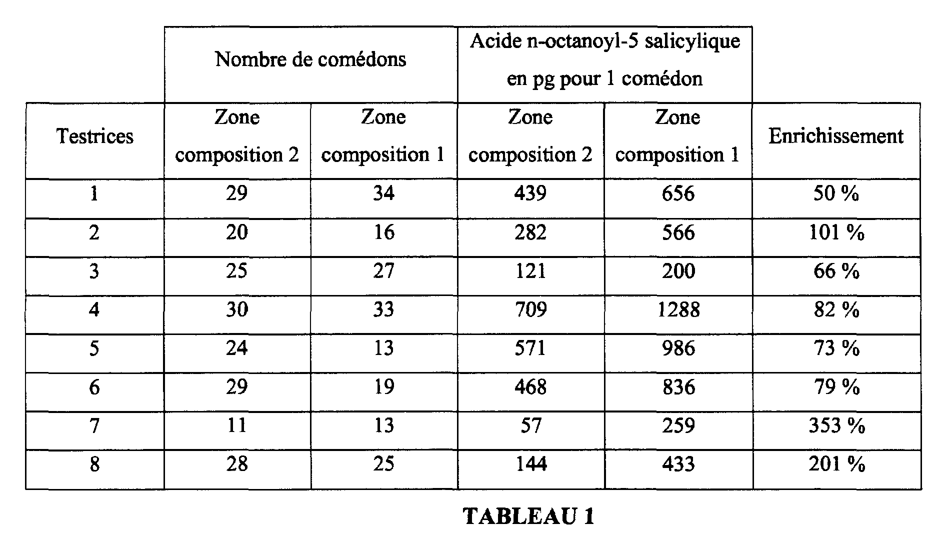

On constate, d'après les résultats exposés ci-dessus, que la composition 1 selon l'invention qui contient les particules poreuses chargées d'acide n-octanoyl-5 salicylique, permet d'augmenter significativement le taux d'acide n-octanoyl-5 salicylique dans le follicule à raison d'au moins 50 % par rapport à une composition sous forme d'émulsion contenant la même quantité d'acide n-octanoyl-5 salicylique.

Cet essai montre l'efficacité des particules poreuses de l'invention pour véhiculer des molécules actives dans l'unité pilo-sébacée.

### Exemple 3: Préparation des particules organiques contenant un composé actif

| Composition des particules | |
|---|---|
| Particules poreuses de Nylon-12 commercialisées sous la dénomination d'« Orgasol 2002 UD Nat Cos »® par la société ATOFINA | 7,5 g |
| Triclosan | 2,5 g |

2,5 g de triclosan sont solubilisés dans 50 ml d'acétone. Dans ce mélange, on introduit 7,5 g d'« Orgasol® ». La dispersion est ensuite introduite dans un évaporateur rotatif afin d'éliminer l'acétone. On obtient alors une poudre chargée en triclosan.

La poudre ainsi obtenue peut être ensuite redispersée dans de l'eau, dans un gel ou dans une émulsion. On veillera à ce que la composition dans laquelle sont introduites les particules contenant le triclosan ne favorise pas la fuite de celui-ci dans le support galénique.

### Exemple 4: Préparation de particules organiques contenant un composé actif

### Composition des particules

| | |
|---|---|
| Particules poreuses de silice commercialisées sous la dénomination de « God Balls2 EC® » par la société SUZUKI OILS & FATS | 7,5 g |
| Vitamine E | 1,5 g |
| Acide n-octanoyl-5 salicylique | 1,0 g |

1,5 g de vitamine E et 1 g d'acide n-octanoyl-5 salicylique sont solubilisés dans 50 ml d'acétone. Dans ce mélange, on introduit 7,5 g de particules poreuses « God Balls 2 EC® ». La dispersion est ensuite introduite dans un évaporateur rotatif afin d'éliminer l'acétone. On obtient alors une poudre chargée en vitamine E et en acide n-octanoyl-5 salicylique.

La poudre ainsi obtenue peut être ensuite redispersée dans de l'eau, dans un gel ou dans une émulsion. On veillera cependant à ce que la composition dans laquelle sont introduites les particules contenant la vitamine E et l'acide n-octanoyl-5 salicylique ne favorise pas la fuite de ceux-ci dans le support galénique.

De façon similaire, on a préparé une poudre de particules avec 7,5 g de particules poreuses « God Balls 2 EC® » et 2,5 g de triclosan. La poudre est observée au microscope électronique. Sa photographie est présentée en figure 1.

On constate que la poudre ainsi obtenue est constituée de particules individualisées.

### Exemple 5 : crème anti-acné (émulsion huile/eau)

| | |
|---|---|
| Polyacryloyldiméthyltaurate d'ammonium | 0,40 g |
| Gomme de xanthane | 0,20 g |
| Conservateurs | 0,80 g |
| EDTA disodique | 0,05 g |
| Glycérol | 5,00 g |
| Eau déminéralisée | 75,04 g |
| Particules poreuses selon l'exemple 3 | 23,00 g |
| Mélange d'alcool cétéarylique/tartrate de dimyristyle/C12-15 Pareth-7/ | |
| PPG-25-Laureth-25 | 1,50 g |
| Alcool stéarylique | 1,00 g |
| Mélange de stéarate de glycéryle / stéarate de PEG-100 | 2,00 g |
| Cyclohexasiloxane | 10,00 g |
| Méthoxycinnamate d'éthylhexyle | 1,00 g |
| Parfum | 0,01 g |

Cette crème douce et fraîche permet de lutter contre les problèmes d'acné avec une bonne efficacité.

### Exemple 6 : lotion tonique

| | |
|---|---|
| Butylène glycol | 1,00 g |
| Oxyde de zinc | 0,50 g |
| Acide lactique | 0,10 g |
| Glycérol | 1,00 g |
| Propylène glycol | 0,20 g |
| PEG-60 huile de ricin hydrogénée | 0,15 g |
| Ethanol | 5,00 g |
| Silice colloïdale 30 nm | 0,50 g |
| Particules poreuses selon l'exemple 3 | 1,00 g |
| Eau déminéralisée | 90,33 g |
| Extrait d'Hamamelis virginiana | 0,0002 g |
| Menthoxypropanediol | 0,01 g |
| Méthylparaben | 0,20 g |
| Parfum | 0,01 g |

### Exemple 7 : émulsion E/H

| **Phase A :** | |
|---|---|
| Isohexadécane | 8,00 g |
| Squalane | 3,70 g |
| Polydiméthylsiloxane (viscosité : 10 cst) | 4,10 g |
| Huile d'amandes d'abricot | 2,30 g |
| Lubrizol 5603 | 1,90 g |

| **Phase B :** | |
|---|---|
| Acide ascorbique | 2,00 g |
| Hydroxyde de potassium à 50 % | 1,20 g |
| Eau déminéralisée | 67,80 g |
| Glycérol | 5,00 g |
| Conservateurs | 1,00 g |

| **Phase C :** | |
|---|---|
| Particules selon l'exemple 3 | 3,00 g |

A température ambiante, on émulsionne lentement la phase B dans la phase A puis on ajoute la phase C.

## Revendications

1. Particules poreuses individualisées **caractérisées en ce qu'**elles présentent un diamètre moyen, en volume, inférieur ou égal à 10 µm et une surface spécifique supérieure ou égale à 1 m²/g, et qu'elles comprennent au moins un composé cosmétiquement ou pharmaceutiquement actif au moins présent à l'intérieur desdites particules.

2. Particules selon la revendication 1, **caractérisées en ce qu'**elles présentent une surface spécifique allant de 2,5 à 1000 m²/g et en particulier de 3 à 750 m²/g.

3. Particules selon l'une quelconque des revendications précédentes, **caractérisées en ce qu'**elles présentent une masse volumique supérieure ou égale à 0,15 g/cm³ et en particulier allant de 0,2 à 5 g/cm³.

4. Particules selon l'une quelconque des revendications précédentes, **caractérisées en ce qu'**elles présentent un diamètre moyen, en volume, supérieur ou égal à 0,1 µm, et en particulier allant de 0,5 à 8 µm.

5. Particules selon l'une quelconque des revendications précédentes, **caractérisées en ce qu'**elles présentent une dimension moyenne en nombre allant d'environ 0,1 à 50 µm, de préférence de 0,1 à 20 µm et tout particulièrement de 0,5 à 10 µm.

6. Particules selon l'une quelconque des revendications précédentes, **caractérisées en ce qu'**elles présentent un indice de polydispersité allant de 1 à 4, et en particulier inférieur ou égal à 3.

7. Particules selon l'une quelconque des revendications précédentes, **caractérisées en ce qu'**elles se présentent sous forme d'une poudre.

8. Particules selon l'une quelconque des revendications précédentes, **caractérisées en ce qu'**elles dérivent de particules poreuses organiques.

9. Particules selon la revendication 8, **caractérisées en ce que** lesdites particules poreuses organiques sont choisies parmi les particules de Nylon 6, Nylon 6-6, Nylon 12 et de Nylon 6-12 et les particules de polyméthacrylate de méthyle.

10. Particules selon l'une quelconque des revendications 1 à 7, **caractérisées en ce qu'**elles dérivent de particules poreuses inorganiques.

11. Particules selon la revendication 10, **caractérisées en ce que** lesdites particules poreuses inorganiques sont choisies parmi les particules de silice, de silice-alumine, d'hydroxyapatite, de dioxyde de titane ou de leurs mélanges.

12. Particules selon l'une quelconque des revendications précédentes, **caractérisées en ce qu'**elles dérivent de particules poreuses en un matériau composite organique et inorganique.

13. Particules selon l'une quelconque des revendications précédentes, **caractérisées en ce que** le rapport pondéral entre le ou les composé(s) actif(s) et les particules poreuses non chargées en composé(s) actif(s) est de 1/1000 à 10/1, et en particulier de 1/100 à 1/1.

14. Particules selon l'une quelconque des revendications précédentes, **caractérisées en ce qu'**elles comprennent au moins un composé cosmétiquement ou pharmaceutiquement actif choisi parmi les agents antibactériens, les agents antifongiques, les agents sébo-régulateurs, les agents sébo-stimulateurs, les agents anti-vieillissement de la peau, notamment les agents kératolytiques ou prodesquamants, les agents de traitement de l'acné, les antibiotiques, les agents inhibiteurs de la chute du cheveu, les agents stimulateurs de la pousse du cheveu, les agents inhibiteurs de la pousse du cheveu ou du poil, les agents anti-pelliculaires, les agents antioxydants, les agents astringents, les agents réducteurs de pores, les agents anti-transpirants, les vitamines, les agents anti-inflammatoires et leurs mélanges.

15. Composition cosmétique ou pharmaceutique **caractérisée en ce qu'**elle comprend des particules telles que définies dans l'une quelconque des revendications 1 à 14.

16. Composition cosmétique ou pharmaceutique selon la revendication 14, **caractérisée en ce que** la proportion en poids desdites particules par rapport au poids total de ladite composition est de 0,1 à 50 % et en particulier de 0,2 à 20 %.

17. Composition cosmétique ou pharmaceutique selon l'une quelconque des revendications 15 et 16, **caractérisée en ce qu'**elle se présente sous la forme d'une lotion, d'une émulsion H/E, d'une émulsion E/H, d'un gel aqueux ou hydroalcoolique ou sous forme anhydre telle qu'un stick, un spray ou une poudre compacte ou libre.

18. Composition cosmétique ou pharmaceutique selon l'une quelconque des revendications 15 et 16, **caractérisée en ce qu'**elle se présente sous la forme d'un produit démaquillant, notamment une huile ou un gel ou une lotion démaquillante ou moussante, ou bien un produit de maquillage tel que fond de teint, crème teintée, mascara ou eye-liner.
